# FASCICULE DE BREVET EUROPEEN

(11) **EP 2 178 461 B1**
(45) Date de publication et mention de la délivrance du brevet: **18.04.2012**
(21) Numéro de dépôt: 08836401.3
(22) Date de dépôt: 09.07.2008
(51) Int. Cl.: A61F 2/14

(54) **LENTILLE DIFFRACTIVE INTRACORNEENNE**
INTRAKORNEALE DIFFRAKTIVE LINSE
INTRACORNEAL DIFFRACTIVE LENS

(30) Priorité: 09.07.2007 FR 0704963
(43) Date de publication de la demande: 28.04.2010
(73) Titulaire: Cohen, Gilbert, 69009 Lyon (FR)
(72) Inventeur: Cohen, Gilbert, 69009 Lyon (FR)
(74) Mandataire: Chevalier, Renaud Philippe
(86) Numéro de dépôt international: PCT/FR2008/001001
(87) Numéro de publication internationale: WO 2009/043985

(56) Documents cités:
- EP-A- 0 420 549
- WO-A-99/07309
- WO-A-02/069849
- US-A- 4 715 858
- US-A- 4 799 931
- US-A- 5 628 794
- US-A1- 2004 243 231

## Description

La présente invention concerne les lentilles diffractives, en particulier les lentilles intracornéennes, qui sont destinées à être implantées dans la cornée pour la correction de défauts de la vision, aussi désignés comme amétropies. Plus particulièrement, cette invention s'intéresse à une lentille diffractive intracornéenne, utilisable pour la correction chirurgicale de la presbytie.

Dans le domaine de la correction des amétropies par chirurgie réfractive, on distingue la chirurgie réfractive cornéenne et la chirurgie endoculaire, la chirurgie cornéenne présentant moins de complications.

La chirurgie réfractive cornéenne est réalisée, à ce jour, par modification de la courbure de la surface antérieure de la cornée.

Plus particulièrement, la correction de la presbytie par la chirurgie cornéenne se fonde sur la pseudo-accommodation, c'est-à-dire sur la transformation de la cornée en dioptre multifocal par modification de la courbure de la cornée ; dans ce mode de correction réfractif, les performances optiques sont dépendantes du diamètre pupillaire, donc du niveau d'illumination.

Dans la correction de la presbytie par la chirurgie endoculaire, l'utilisation de lentilles diffractives donne de bons résultats, indépendants du centrage de la lentille et du diamètre pupillaire.

La transformation de la cornée en une lentille diffractive par sculpture n'est pas possible. Seule l'utilisation d'une lentille diffractive intracornéenne permettrait de bénéficier des propriétés optiques des lentilles diffractives et de l'innocuité de la chirurgie cornéenne.

Les obstacles actuels à l'utilisation d'implants intracornéens, en particulier de lentilles diffractives intracornéennes, notamment pour le traitement de la presbytie, sont la biocompatibilité de ces implants et surtout leur perméabilité aux flux de nutriments et d'oxygène dans l'épaisseur de la cornée, perméabilité qui est indispensable au maintien de la transparence et de la fonction réfractive de la cornée.

Les hydrogels à forte teneur en eau sont certes perméables aux nutriments et à l'oxygène, mais ils possèdent un indice de réfraction optique proche de celui de la cornée et sont donc sans efficacité optique pour la réalisation de lentilles diffractives intracornéennes.

Les documents EP 0420549 A2 et WO 99/07309 montrent des exemples de lentilles cornéennes, réalisées à partir d'hydrogels et comportant des zones annulaires concentriques, disposées en gradins. Ces documents permettent de comprendre que, si l'un des deux composants de la lentille n'est pas un hydrogel perméable, il forme une couche continue faisant barrage au flux de nutriments et d'oxygène.

La présente invention vise à résoudre les problèmes ici présentés, et elle a donc pour but de fournir une lentille diffractive intracornéenne, adaptée au traitement de la presbytie et conçue de manière à permettre une bonne circulation des flux de nutriments et d'oxygène dans l'épaisseur de la cornée, lorsque la lentille est implantée, tout en étant manipulable.

A cet effet, l'invention a pour objet une lentille diffractive zonale avec inversion de phase, avec alternance de zones annulaires optiquement actives dites « pleines » et de zones annulaires optiquement inactives dites « vides », toutes ces zones annulaires étant concentriques ou coaxiales, cette lentille étant essentiellement caractérisée par le fait que les zones annulaires « vides » sont occupées par un « ciment » optiquement inactif qui relie entre elles les zones annulaires « pleines », pour assurer la stabilité de ces zones annulaires « pleines ».

Plus particulièrement, la lentille diffractive de l'invention est conçue comme une lentille intracornéenne, dans laquelle le « ciment » des zones annulaires inactives ou « vides » possède une perméabilité aux nutriments et à l'oxygène qui est comparable à celle du tissu cornéen, et un indice optique proche de celui de la cornée.

Les zones annulaires « pleines » d'une telle lentille peuvent présenter, par rapport aux zones annulaires « vides », une différence d'indice optique telle que l'indice optique des zones annulaires « pleines » soit :
- ou bien plus fort que celui des zones annulaires « vides »;
- ou bien, en variante, plus faible que celui des zones annulaires « vides ».

Dans une forme de réalisation préférée de la lentille diffractive intracornéenne, objet de l'invention, les zones annulaires « vides » sont remplies par un hydrogel à forte teneur en eau, perméable mais optiquement inactif, constituant le « ciment » reliant les zones annulaires « pleines » pour le maintien de la répartition spatiale concentrique ou coaxiale de ces zones annulaires « pleines », et qui facilite ainsi la manipulation de la lentille. L'hydrogel qui sert ici de « ciment » reliant les zones annulaires « pleines » est en particulier un hydrogel dont le pourcentage en eau est égal ou supérieur à 78 %. Les zones annulaires « pleines », peuvent aussi être réalisées en hydrogel, dont le pourcentage en eau est :
- soit inférieur à 78 %, et de préférence compris entre 50 et 70 % ;
- soit supérieur à 78 %, et de préférence supérieur à 85%, voire être constituées par de l'eau.

Ainsi, la lentille diffractive intracornéenne, objet de l'invention, se caractérise par une alternance d'anneaux concentriques « pleins », réalisés dans une matière choisie pour son indice optique, et d'anneaux « vides » de préférence remplis d'hydrogel perméable qui assure la cohérence de l'ensemble, les zones « vides » remplies d'hydrogel étant perméables aux nutriments et à l'oxygène, et leur alternance régulière et rapprochée permettant une bonne circulation des flux dans l'épaisseur de la cornée.

La géométrie d'une telle lentille diffractive zonale, dont les parties optiquement actives sont des zones annulaires « pleines » et concentriques séparées par des interstices, est justifiée de manière théorique, par le principe des lentilles de Fresnel et par la notion d'inversion de phase (Rayleigh Wood phase reversal zone plate).

En son centre, cette lentille peut comprendre un disque profilé réalisé dans la même matière que les zones annulaires « pleines », et entouré concentriquement ou coaxialement par ces zones annulaires « pleines », le disque central constituant une zone optiquement active à la manière d'un premier anneau de rayon intérieur nul. En variante, la lentille comprend en son centre une zone circulaire « vide », donc optiquement inactive, qui est entourée coaxialement par la première zone annulaire « pleine ».

Pour des raisons de fabrication, les zones annulaires « pleines » de la lentille, et le cas échéant le disque central, peuvent être reliés par une fine membrane réalisée dans la même matière optiquement active, ladite membrane restant perméable aux nutriments en raison de sa très faible épaisseur.

Dans une autre forme de réalisation, et toujours pour des raisons de fabrication, les zones annulaires « pleines » de la lentille, et le cas échéant le disque central, sont reliés par des ponts de matière d'orientation générale radiale, réalisés dans la même matière optiquement active, ces ponts de matière traversant les zones annulaires « vides ».

La membrane ou les ponts de matière facilitent en particulier la manipulation des anneaux, et/ou servent de canaux d'injection lors de la fabrication, sans modifier les propriétés optiques de la lentille ni faire obstacle aux transferts de nutriments.

La lentille diffractive intracornéenne, objet de l'invention, est réalisable comme une lentille monofocale adaptée pour la correction des amétropies sphériques, ou comme une lentille bifocale, cette dernière version étant adaptée à la correction de la presbytie. La définition précise de la géométrie des faces antérieure et postérieure des zones annulaires « pleines » d'une telle lentille contribue à son adaptation à chaque cas particulier d'application. De plus, dans la mesure où les zones annulaires « pleines » sont non seulement reliées par les parties en hydrogel perméable, mais sont aussi enrobées par des parties réalisées dans le même hydrogel perméable dont les surfaces antérieure et postérieure peuvent être parallèles ou non, ces parties en hydrogel peuvent ou non avoir un effet réfractif additionnel.

L'invention sera mieux comprise, et d'autres caractéristiques seront mises en évidence, à l'aide de la description qui suit, en référence au dessin schématique annexé représentant, à titre d'exemples, quelques formes d'exécution de cette lentille diffractive intracornéenne :
Figure 1 est une vue en coupe diamétrale d'une lentille diffractive intracornéenne conforme à la présente invention, dans un premier mode de réalisation ;
Figure 2 est une vue en coupe diamétrale d'une lentille diffractive intracoméenne conforme à la présente invention, dans un deuxième mode de réalisation ;
Figure 3 est une vue similaire à la figure 1, illustrant une variante de la lentille selon le premier mode de réalisation ;
Figure 4 est une vue similaire à la figure 2, illustrant une variante de la lentille selon le deuxième mode de réalisation ;
Figure 5 est une vue de face d'une lentille diffractive intra cornéenne selon un dernier mode de réalisation de l'invention.

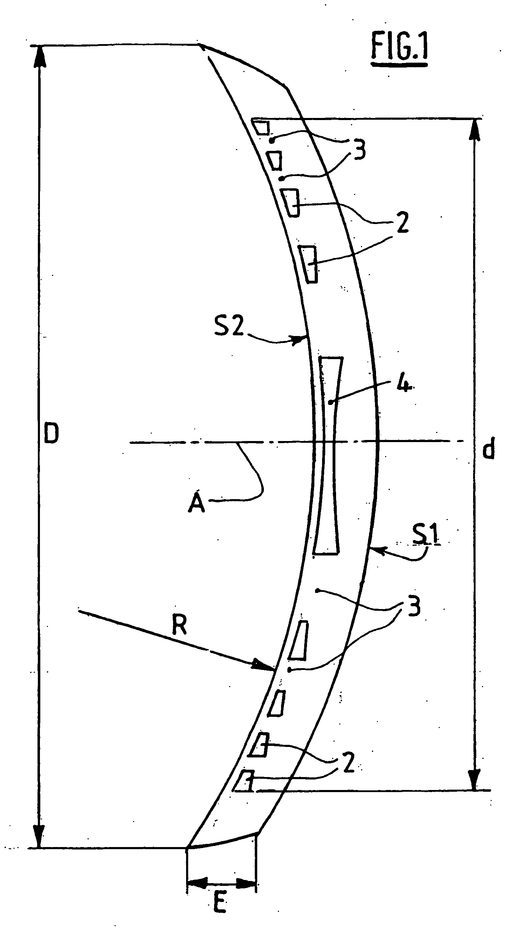

En se référant à la figure 1, une lentille diffractive intracornéenne dont l'axe central est désigné par A possède un diamètre extérieur D pouvant être compris entre 5 et 9 mm, et une courbure moyenne définie par un rayon R pouvant être compris entre 7 et 9 mm. Cette lentille présente une surface extérieure convexe S1 et une surface intérieure concave S2, son épaisseur E mesurée entre les deux surfaces S1 et S2 pouvant être comprise entre 0,05 mm et 0,5 mm.

La zone utile de la lentille, centrée sur l'axe A, est un cercle dont le diamètre d peut être compris entre 3 et 7 mm, selon le diamètre extérieur D de cette lentille. Cette zone utile comprend une succession d'anneaux « pleins » 2 en matière optiquement active, de diamètres croissants, tous centrés sur l'axe A, qui sont séparés les uns des autres par des zones annulaires intermédiaires « vides » 3. Les anneaux « pleins » 2 et les zones intermédiaires « vides » 3 sont de largeur régulièrement décroissante, depuis l'axe central A en direction de la périphérie de la lentille, la géométrie des anneaux « pleins » 2 étant conforme au principe de la lentille zonale de Fresnel. Dans le mode de réalisation de la figure 1, la lentille intracoméenne comprend encore, en son centre, un disque 4 profilé réalisé dans la même matière optiquement active que les anneaux « pleins » 2, et entouré concentriquement ou coaxialement par ces anneaux « pleins » 2. Le disque central 4 peut être assimilé à un premier anneau « plein », de rayon intérieur égal à zéro.

Les zones intermédiaires « vides » 3 sont, en réalité, remplies d'une matière optiquement inactive ou faiblement active, qui est notamment un hydrogel dont le pourcentage en eau est égal ou supérieur à 78 %. Il peut s'agir d'un hydrogel de type acrylate ou métacrylate, acrylamide ou métacylamide, polyester, vinyle copomylère, ou analogue... Cet hydrogel est non seulement présent entre les anneaux « pleins » 2, mais il peut aussi enrober entièrement ces anneaux « pleins » 2, ainsi que le disque central 4, en s'étendant jusqu'aux surfaces extérieure S1 et intérieure S2. Dans tous les cas, cet hydrogel forme un « ciment » qui relie tous les anneaux 2 entre eux, stabilisant ainsi la structure de la lentille.

Les anneaux « pleins » 2 et le disque central 4 sont réalisés dans une matière possédant un indice optique différent de celui de la cornée. Il peut ici s'agir également d'un hydrogel, mais dont le pourcentage en eau est inférieur à 78 %, et de préférence compris entre 50 % et 70%.

Les anneaux « pleins » 2, dont le nombre peut être compris entre cinq et trente (le dessin représentant de façon simplifiée un très faible nombre d'anneaux), sont de perméabilité inférieure à celle de la cornée et provoquent, avec le disque central 4, la diffraction nécessaire à la correction de vision souhaitée.

Les zones annulaires intermédiaires « vides » 3, remplies d'hydrogel, réalisent la liaison entre les anneaux 2 tout en étant perméables aux flux de nutriments et à l'oxygène.

Les surfaces extérieure S1 et intérieure S2 peuvent être parallèles, donc sans effet sur la correction réalisée, ou au contraire être non parallèles et conformées de manière à participer à la correction visuelle, par un effet réfractif additionnel.

Une telle lentille diffractive intracornéenne, associant deux matières, est réalisable par des techniques de moulage ou de surmoulage. En particulier, elle peut être fabriquée pour un procédé de double injection.

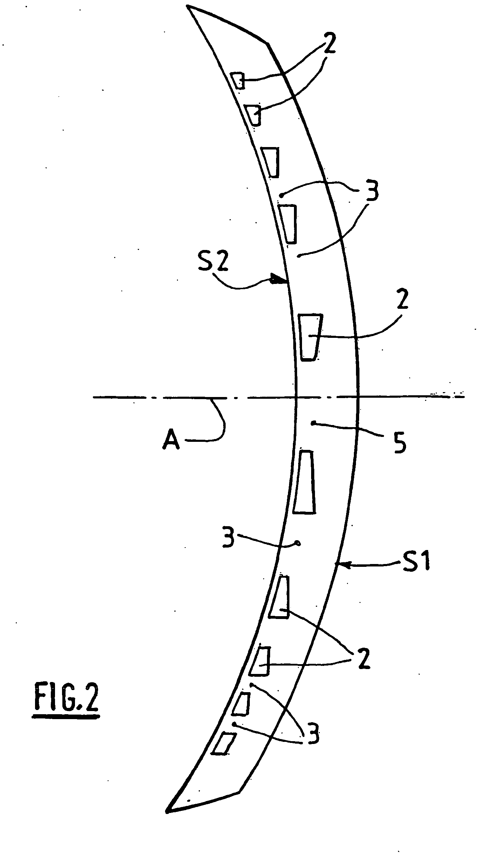

La figure 2, sur laquelle les éléments correspondant à ceux précédemment décrits sont désignés par les mêmes références (lettres ou chiffres), représente une variante de cette lentille diffractive intracornéenne. Dans cette variante, le disque central est supprimé. La lentille comprend donc en son centre une zone circulaire « vide » 5, ou remplie de matière optiquement inactive ou faiblement active mais perméable, telle qu'un hydrogel adapté ; la zone circulaire centrale 5 est entourée concentriquement par la première zone annulaire « pleine », c'est-à-dire par le premier anneau 2.

Dans une variante, non illustrée directement au dessin, de cette lentille diffractive intracornéenne, les anneaux 2 possèdent un indice optique plus faible que celui du « ciment » qui relie ces anneaux. Dans ce cas le « ciment » reste, en particulier, un hydrogel dont la teneur en eau est proche de 78 %, tandis que les anneaux 2 sont réalisés dans un hydrogel dont la teneur en eau est plus élevée que celle dudit « ciment », et typiquement supérieure à 85 %, voire constitués par de l'eau.

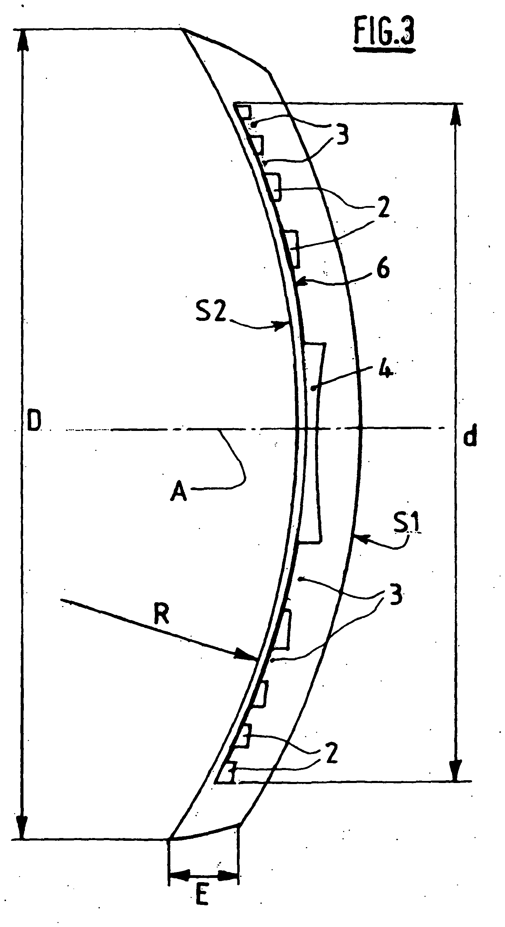

La figure 3 illustre une variante de la lentille selon la figure 1, dans laquelle les anneaux « pleins » 2, ainsi que le disque central 4, sont reliés les uns aux autres par une fine membrane 6 réalisée dans la même matière optiquement active. La membrane 6, noyée ici dans les zones annulaires intermédiaires « vides » 3, reste perméable aux nutriments en raison de sa très faible épaisseur, et elle permet ainsi aux zones annulaires « vides » 3 de jouer encore leur rôle.

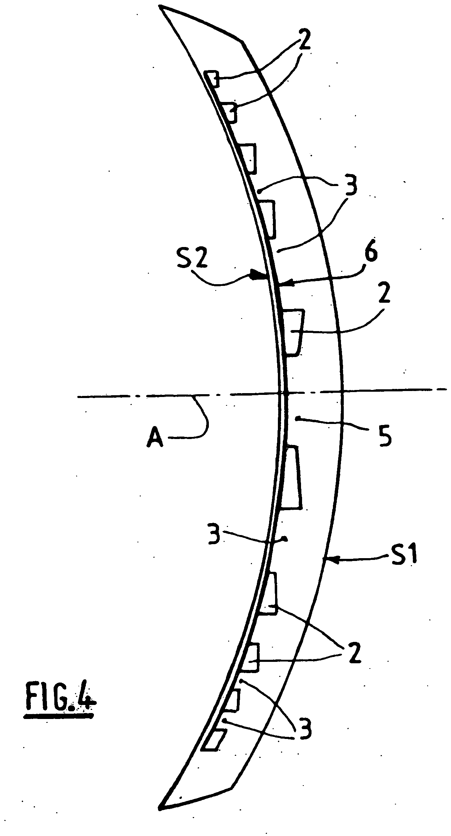

De manière analogue, la figure 4 illustre une variante de la lentille selon la figure 2, dans la quelle les anneaux « pleins » 2 sont reliés les uns aux autres par une fine membrane 6 réalisée dans la même matière optiquement active. En l'absence de disque central, la membrane 6 est ici présente dans les zones annulaires intermédiaires « vides » 3, ainsi que dans la zone circulaire centrale 5, et comme précédemment elle ne fait pas obstacle aux transferts de nutriments.

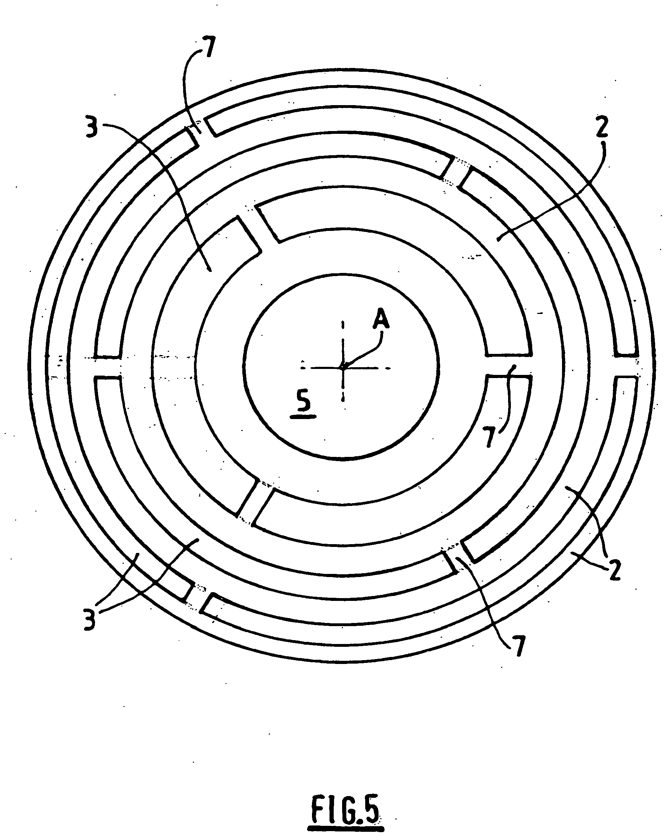

Enfin, la figure 5 représente une autre forme de réalisation, dans laquelle les anneaux « pleins » 2 de la lentille sont reliés entre eux par des ponts de matière 7 de direction radiale, réalisés dans la même matière optiquement active que ces anneaux 2. En raison de leur finesse, les ponts de matière 7 ménagent entre eux et les anneaux 2 de larges espaces en forme arcs de cercle remplis par la matière inactive mais perméable des zones intermédiaires « vides » 3.

Comme on le comprend, la présence de la membrane 6 ou des ponts de matière 7 facilite la fabrication de la lentille, sans gêner la vision et sans altérer la perméabilité des zones annulaires intermédiaires « vides » 3.

On ne s'éloignerait pas du cadre de l'invention, telle que définie dans les revendications annexées, quels que soient notamment :
- les dimensions de la lentille ;
- la nature de ses matières constitutives ;
- le nombre de ses anneaux pleins ;
- la nature du défaut de la vision corrigé par cette lentille.

## Revendications

1. Lentille diffractive zonale avec inversion de phase, avec alternance de zones annulaires optiquement actives dites « pleines » (2) et de zones annulaires optiquement inactives dites « vides » (3), toutes ces zones annulaires étant concentriques ou coaxiales, **caractérisée en ce que** les zones annulaires « vides » (3) sont occupées par un « ciment » optiquement inactif qui relie entre elles les zones annulaires « pleines » (2), pour assurer la stabilité de ces zones annulaires « pleines » (2).

2. Lentille diffractive selon la revendication 1, conçue comme une lentille intracornéenne, **caractérisée en ce que** le « ciment » des zones annulaires inactives ou « vides » (3) possède une perméabilité aux nutriments et à l'oxygène qui est comparable à celle du tissu cornéen, et un indice optique proche de celui de la cornée.

3. Lentille difractive intracoméenne selon la revendication 2, **caractérisée en ce que** les zones annulaires « pleines » (2) présentent, par rapport aux zones annulaires vides (3), une différence d'indice optique telle que l'indice optique des zones annulaires pleines (2) soit plus fort que celui des zones annulaires vides (3).

4. Lentille difractive intracoméenne selon la revendication 2, **caractérisée en ce que** les zones annulaires « pleines » (2) présentent, par rapport aux zones annulaires vides (3), une différence d'indice optique telle que l'indice optique des zones annulaires pleines (2) soit plus faible que celui des zones annulaires vides (3).

5. Lentille diffractive intracornéenne selon la revendication 3 ou 4, **caractérisée en ce que** les zones annulaires « vides » (3) sont remplies par un hydrogel à forte teneur en eau, perméable mais optiquement inactif, constituant le « ciment » reliant entre elles les zones annulaires « pleines » (2).

6. Lentille diffractive intracoméenne selon la revendication 5, **caractérisée en ce que** l'hydrogel qui sert de « ciment » reliant les zones annulaires
« pleines » (2) est un hydrogel dont le pourcentage en eau est égal ou supérieur à 78 %.

7. Lentille diffractive intracoméenne selon la revendication 6, **caractérisée en ce que** les zones annulaires « pleines » (2) sont également en hydrogel, dont le pourcentage en eau est inférieur à 78 %, et de préférence compris entre 50 et 70 %.

8. Lentille diffractive intracornéenne selon la revendication 6, **caractérisée en ce que** les zones annulaires « pleines » (2) sont également en hydrogel, dont le pourcentage en eau est supérieur à 78 %, et de préférence supérieur à 85 %, voire constituées d'eau.

9. Lentille diffractive intracoméenne selon l'une des revendications 2 à 8, **caractérisée en ce que** les zones annulaires « pleines » (2) sont non seulement reliées par les parties en hydrogel perméable, mais sont aussi enrobées par des parties réalisées dans le même hydrogel.

10. Lentille diffractive intracoméenne selon l'une des revendications 1 à 9, **caractérisée en ce qu'**elle comprend en son centre un disque (4) profilé réalisé dans la même matière active que les zones annulaires « pleines » (2), et entouré concentriquement ou coaxialement par ces zones annulaires « pleines » (2), le disque central (4) constituant une zone optiquement active à la manière d'un premier anneau de rayon intérieur nul.

11. Lentille diffractive intracoméenne selon l'une des revendications 1 à 9, **caractérisé en ce qu'**elle comprend en son centre une zone circulaire « vide » (5), donc optiquement inactive, qui est entourée concentriquement par la première zone annulaire « pleine » (2).

12. Lentille diffractive intracornéenne selon la revendication 10 ou 11, **caractérisée en ce que** les zones annulaires « pleines » (2), et le cas échéant le disque central (4), sont reliés par une fine membrane (6) réalisée dans la même matière optiquement active, ladite membrane (6) restant perméable aux nutriments en raison de sa très faible épaisseur.

13. Lentille diffractive intracoméenne selon la revendication 10 ou 11, **caractérisée en ce que** les zones annulaires « pleines » (2), et le cas échéant le disque central (4), sont reliés par des ponts de matière (7) d'orientation générale radiale, réalisés dans la même matière optiquement active, ces ponts de matière (7) traversant le zones annulaires « vides » (3).

14. Lentille diffractive intracoméenne selon l'une des revendications 1 à 13, **caractérisée en ce qu'**elle est réalisée comme une lentille bifocale adaptée à la correction de la presbytie.

## Claims

1. A zonal diffractive lens with phase reversal and with an alternation of optically active or "full" annular zones (2) and of optically inactive or "empty" annular zones (3), all of these annular zones being concentric or coaxial, **characterized in that** the "empty" annular zones (3) are occupied by an optically inactive "cement" that interconnects the "full" annular zones (2) in order to ensure the stability of these "full" annular zones (2).

2. The diffractive lens as claimed in claim 1, designed as an intracorneal lens, **characterized in that** the "cement" of the inactive or "empty" annular zones (3) has a permeability to nutrients and to oxygen that is comparable to the permeability of the corneal tissue and has an optical index close to that of the cornea.

3. The intracorneal diffractive lens as claimed in claim 2, **characterized in that** the "full" annular zones (2) have a different optical index compared to the empty annular zones (3), such that the optical index of the full annular zones (2) is greater than that of the empty annular zones (3).

4. The intracorneal diffractive lens as claimed in claim 2, **characterized in that** the "full" annular zones (2) have a different optical index compared to the empty annular zones (3), such that the optical index of the full annular zones (2) is less than that of the empty annular zones (3).

5. The intracorneal diffractive lens as claimed in claim 3 or 4, **characterized in that** the "empty" annular zones (3) are filled by a hydrogel which has a high water content and which is permeable but optically inactive and constitutes the "cement" interconnecting the "full" annular zones (2).

6. The intracorneal diffractive lens as claimed in claim 5, **characterized in that** the hydrogel that serves as a "cement" connecting the "full" annular zones (2) is a hydrogel whose percentage of water is equal to or greater than 78%.

7. The intracorneal diffractive lens as claimed in claim 6, **characterized in that** the "full" annular zones (2) are also made of a hydrogel whose percentage of water is less than 78%, preferably between 50 and 70%.

8. The intracorneal diffractive lens as claimed in claim 6, **characterized in that** the "full" annular zones (2) are also made of a hydrogel whose percentage of water is greater than 78%, preferably greater than 85%, or are even formed by water.

9. The intracorneal diffractive lens as claimed in one of claims 2 to 8, **characterized in that** the "full" annular zones (2) are not only connected by the parts made of permeable hydrogel but are also coated by parts made of the same hydrogel.

10. The intracorneal diffractive lens as claimed in one of claims 1 to 9, **characterized in that** it comprises, at its center, a profiled disk (4) made of the same active material as the "full" annular zones (2) and surrounded concentrically or coaxially by these "full" annular zones (2), the central disk (4) constituting an optically active zone in the manner of a first ring with an inner radius of zero.

11. The intracorneal diffractive lens as claimed in one of claims 1 to 9, **characterized in that** it comprises, at its center, an "empty" and therefore optically inactive circular zone (5), which is surrounded concentrically by the first "full" annular zone (2).

12. The intracorneal diffractive lens as claimed in claim 10 or 11, **characterized in that** the "full" annular zones (2), and if appropriate the central disk (4), are connected by a fine membrane (6) made of the same optically active material, said membrane (6) remaining permeable to nutrients because of its very slight thickness.

13. The intracorneal diffractive lens as claimed in claim 10 or 11, **characterized in that** the "full" annular zones (2), and if appropriate the central disk (4), are connected by material bridges (7) whose general orientation is radial and which are made of the same optically active material, said material bridges (7) extending across the "empty" annular zones (3).

14. The intracorneal diffractive lens as claimed in one of claims 1 to 13, **characterized in that** it is produced as a bifocal lens designed to correct presbyopia.

## Patentansprüche

1. Zonale diffraktive Linse mit Phasenumkehr mit einem Wechsel zwischen optisch aktiven ringförmigen Zonen, genannt "voll" (2), und optisch inaktiven ringförmigen Zonen, genannt "leer" (3), wobei alle diese ringförmigen Zonen konzentrisch oder koaxial sind, **dadurch gekennzeichnet, dass** die "leeren" ringförmigen Zonen (3) von einem optisch inaktiven "Bindemittel" besetzt sind, das die "vollen" (2) ringförmigen Zonen untereinander verbindet, um die Stabilität dieser vollen (2) ringförmigen Zonen sicherzustellen.

2. Diffraktive Linse nach Anspruch 1, entworfen wie eine intrakorneale Linse, **dadurch gekennzeichnet, dass** das "Bindemittel" der inaktiven oder "leeren" (3) ringförmigen Zonen eine Durchlässigkeit für Nährstoffe und für Sauerstoff aufweist, die mit derjenigen des Hornhautgewebes vergleichbar ist, sowie einen optischen Index, der nahe demjenigen der Hornhaut liegt.

3. Intrakorneale diffraktive Linse nach Anspruch 2, **dadurch gekennzeichnet, dass** die "vollen" (2) ringförmigen Zonen in Bezug auf die leeren (3) ringförmigen Zonen einen derartigen Unterschied des optischen Indexes aufweisen, dass der optische Index der vollen (2) ringförmigen Zonen stärker als derjenige der leeren (3) ringförmigen Zonen ist.

4. Intrakorneale diffraktive Linse nach Anspruch 2, **dadurch gekennzeichnet, dass** die "vollen" (2) ringförmigen Zonen in Bezug auf die leeren (3) ringförmigen Zonen einen derartigen Unterschied des optischen Indexes aufweisen, dass der optische Index der vollen (2) ringförmigen Zonen schwächer als derjenige der leeren (3) ringförmigen Zonen ist.

5. Intrakorneale diffraktive Linse nach Anspruch 3 oder 4, **dadurch gekennzeichnet, dass** die "leeren" (3) ringförmigen Zonen mit einem Hydrogel mit einem hohen Wassergehalt gefüllt sind, das durchlässig aber optisch inaktiv ist, das das ·"Bindemittel" darstellt, das die "vollen" (2) ringförmigen Zonen miteinander verbindet.

6. Intrakorneale diffraktive Linse nach Anspruch 5, **dadurch gekennzeichnet, dass** das Hydrogel, das als "Bindemittel" dient, das die "vollen" (2) ringförmigen Zonen verbindet, ein Hydrogel ist, dessen Prozentsatz an Wasser gleich oder höher als 78 % ist.

7. Intrakorneale diffraktive Linse nach Anspruch 6, **dadurch gekennzeichnet, dass** die "vollen" (2) ringförmigen Zonen ebenfalls aus Hydrogel sind, dessen Prozentsatz an Wasser geringer als 78 % ist und vorzugsweise zwischen 50 und 70 % liegt.

8. Intrakorneale diffraktive Linse nach Anspruch 6, **dadurch gekennzeichnet, dass** die "vollen" (2) ringförmigen Zonen ebenfalls aus Hydrogel sind, dessen Prozentsatz an Wasser höher als 78 % und vorzugsweise höher als 85 % ist, ja sogar aus Wasser bestehen.

9. Intrakorneale diffraktive Linse nach einem der Ansprüche 2 bis 6, **dadurch gekennzeichnet, dass** die "vollen" (2) ringförmigen Zonen nicht nur durch die Teile aus durchlässigem Hydrogel verbunden sind, sondern auch mit Teilen umhüllt sind, die aus dem gleichen Hydrogel hergestellt sind.

10. Intrakorneale diffraktive Linse nach einem der Ansprüche 1 bis 9, **dadurch gekennzeichnet, dass** sie in ihrer Mitte eine profilierte Scheibe (4) umfasst, die aus dem gleichen aktiven Material wie die "vollen" (2) ringförmigen Zonen hergestellt ist, und konzentrisch oder koaxial von diesen "vollen" (2) ringförmigen Zonen umgeben ist, wobei die mittlere Scheibe (4) eine optisch aktive Zone nach Art eines ersten Rings mit einem Innenradius gleich null darstellt.

11. Intrakorneale diffraktive Linse nach einem der Ansprüche 1 bis 9, **dadurch gekennzeichnet, dass** sie in ihrer Mitte eine "leere" (5) und somit optisch inaktive ringförmige Zone umfasst, die konzentrisch von der ersten "vollen" (2) ringförmigen Zone umgeben ist.

12. Intrakorneale diffraktive Linse nach Anspruch 10 oder 11, **dadurch gekennzeichnet, dass** die "vollen" (2) ringförmigen Zonen und gegebenenfalls die mittlere Scheibe (4) durch eine feine Membran (6) verbunden sind, die aus dem gleichen optisch aktiven Material hergestellt ist, wobei die Membran (6) aufgrund ihrer sehr geringen Dicke für Nährstoffe durchlässig bleibt

13. Intrakorneale diffraktive Linse nach Anspruch 10 oder 11, **dadurch gekennzeichnet, dass** die "vollen" (2) ringförmigen Zonen und gegebenenfalls die mittlere Scheibe (4) durch Materialbrücken (7) mit einer im Allgemeinen radialen Ausrichtung verbunden sind, die aus dem gleichen optisch aktiven Material hergestellt sind, wobei diese Materialbrücken (7) die "leeren" (3) ringförmigen Zonen durchqueren.

14. Intrakorneale diffraktive Linse nach einem der Ansprüche 1 bis 13, **dadurch gekennzeichnet, dass** sie wie eine bifokale Linse hergestellt ist, die für die Korrektur der Presbyopie ausgelegt ist.
